**Europäisches Patentamt**

**European Patent Office**

**Office européen des brevets**

(19)

(11) Veröffentlichungsnummer: **0 025 891
B1**

(12) **EUROPÄISCHE PATENTSCHRIFT**

(45) Veröffentlichungstag der Patentschrift:
**30.11.83**

(21) Anmeldenummer: **80105117.8**

(22) Anmeldetag: **28.08.80**

(51) Int. Cl.³: **C 07 C 35/00, C 07 C 35/06,
C 07 C 35/08, C 07 C 35/14,
C 07 C 35/20, C 07 C 35/22,
C 07 C 29/03**

(54) Verfahren zur Hydroxylierung von cyclischen Olefinen mit ein oder zwei Doppelbindungen.

(30) Priorität: **19.09.79 DE 2937810**

(43) Veröffentlichungstag der Anmeldung:
**01.04.81 Patentblatt 81/13**

(45) Bekanntmachung des Hinweises auf die Patenterteilung:
**30.11.83 Patentblatt 83/48**

(84) Benannte Vertragsstaaten:
**AT BE CH FR GB IT LI NL SE**

(56) Entgegenhaltungen:
**DE - B - 1 543 432
DE - C - 574 838
DE - C - 956 505
DE - C - 956 805
GB - A - 643 118
US - A - 3 026 357
US - P - 25 559 27**

**A. Roebuck and H. Adkins, org. Synth., Cole, Vol. III, 1955,
p. 217-219
A. Roebuck and H. Adkins, J. Am. Chem. Soc, 1948, 70 p.
4041 to 4044**

(73) Patentinhaber: **Degussa Aktiengesellschaft,
Weissfrauenstrasse 9, D-6000 Frankfurt am Main 1 (DE)**

(72) Erfinder: **Käbisch, Gerhard, Dr., Palmstrasse 1,
D-7888 Rheinfelden (DE)**
Erfinder: **Malitius, Horst, Blümleacker 5,
D-7888 Rheinfelden (DE)**
Erfinder: **Raupach, Siegfried, Langentalstrasse 24,
D-7888 Rheinfelden (DE)**
Erfinder: **Trübe, Rudolf, Ernst-Reuter-Strasse 22,
D-7888 Rheinfelden (DE)**
Erfinder: **Wittmann, Hans, Katzenbuckelweg 12,
D-7888 Rheinfelden (DE)**

**0 025 891**

## Verfahren zur Hydroxylierung von cyclischen Olefinen mit ein oder zwei Doppelbindungen

Die Erfindung betrifft ein Verfahren zur Hydroxylierung von Cyclopenten, Cyclohexen, Cyclohepten, Bicyclo-(2,2,1)-heptadien-2,5 oder der cyclischen Dimerisierungsprodukte von Butadien oder Isopren, sowie deren Isomerisierungsprodukte durch Umsetzung mit Ameisensäure mit einer Konzentration über 20 Gewichtsprozent und Wasserstoffperoxid mit einer Konzentration von weniger als 50 Gewichtsprozent und in einer Menge von weniger als 2 Mol pro Mol zu hydroxylierender Doppelbindung bei einer Temperatur zwischen 40 und 80° C.

Unter Hydroxylierung ist zu verstehen, daß an die olefinischen Doppelbindungen jeweils zwei Hydroxylgruppen angelagert werden. Bei der Hydroxylierung der Monoolefine entstehen demnach vicinale Diole, bei der Hydroxylierung der Diolefine entsprechend Tetrole.

Durch A. Roebuck and H. Adkins in Org. Synth., Coll. Vol. III, 1955, 217 bis 219, bzw. in J. Am. Chem. Soc., 1948, 70, 4041 bis 4044, ist es bereits bekannt, Cyclohexen bei einer Temperatur zwischen 40 und 45°C mit 13,7 Mol 88%iger Ameisensäure und 1,4 Mol 30%igem Wasserstoffperoxid pro Mol eingesetztem Cyclohexen zu 1,2-Cyclohexandiol zu hydroxylieren. Es wird ausdrücklich erwähnt, daß zwar weniger Ameisensäure verwendet werden kann, daß dann aber die Ausbeuten geringer sind und die Umsetzung nicht so leicht kontrollierbar ist.

Auch aus der GB-A-634 118 ist bereits die Hydroxylierung von cyclischen Olefinen mit ein oder zwei Doppelbindungen mittels Ameisensäure und Wasserstoffperoxid grundsätzlich bekannt. Als geeignete Olefine werden zwar unter anderem Cyclopenten und Cyclohexen genannt, speziell beschrieben ist jedoch nur die Hydroxylierung von Allylalkohol zu Glycerin. Es wird mehrmals empfohlen, auf ein Mol Olefin etwa 1 Mol Ameisensäure zu verwenden. Aus den konkreten Beispielen ergibt sich aber, daß unter diesen Bedingungen eine untragbar lange Reaktionszeit erforderlich ist, die nur durch eine beträchtliche Erhöhung der Ameisensäuremenge bei im wesentlichen gleichem Umsatz und gleicher Ausbeute auf ein vernünftiges Maß vermindert werden kann.

Aus der DE-C-956 505 ist es ferner bereits bekannt, Tricyclo-(5,2,1,0)-decen-(3)-ol-(8) bzw. -(9) bei einer Temperatur von 45°C mit etwa 11,2 Mol 88%iger Ameisensäure und etwa 1,2 Mol 30%igem Wasserstoffperoxid pro Mol eingesetztem Olefin zu den entsprechenden gesättigten Triolestern umzusetzen, die dann anschließend mit wäßrigen Alkalien zu den freien Triolen verseift werden.

Auch in der US-A-2 555 927 wird erläutert, daß bei der Hydroxylierung von olefinisch ungesättigten Verbindungen mit Ameisensäure und Wasserstoffperoxid in hohem Maße eine Veresterung des gewünschten Produktes auftritt. Es wird deshalb ein neues Verfahren vorgeschlagen, bei dem anstelle der Ameisensäure niedrigsiedende Alkylformiate eingesetzt werden.

Das erfindungsgemäße Verfahren ist nun dadurch gekennzeichnet, daß man das zu hydroxylierende Olefin mit weniger als 2 Mol Ameisensäure pro Mol zu hydroxylierender Doppelbindung und dem Wasserstoffperoxid umsetzt, Ameisensäure mit einer Konzentration zwischen 20 und 100 Gewichtsprozent einsetzt, und die Konzentration des Wasserstoffperoxids in der wäßrigen Phase des Reaktionsgemisches während der Gesamtdauer der Umsetzung unter 15 Gewichtsprozent hält.

Vorzugsweise wird die Umsetzung bei einer Temperatur zwischen 45 und 60° C vorgenommen. Die Ameisensäure wird vorteilhafterweise in einer Menge von 0,2 bis 0,8 Mol pro Mol zu hydroxylierender Doppelbindung, das Wasserstoffperoxid vorteilhafterweise in einer Menge von 1,1 bis 1,5 Mol pro Mol zu hydroxylierender Doppelbindung eingesetzt. Bevorzugt wird ein Wasserstoffperoxid mit einer Konzentration von 20 bis 40 Gewichtsprozent verwendet.

Durch das erfindungsgemäße Verfahren können die monocyclischen Monoolefine mit 5 bis 7 Kohlenstoffatomen, das Bicyclo-(2,2,1)-heptadien-2,5, die cyclischen Dimerisierungsprodukte von Butadien oder Isopren, wie Cyclooctadien-1,5 oder 1,5-Dimethylcyclooctadien-1,5, sowie deren Isomerisierungsprodukte, beispielsweise Cyclooctadien-1,3 oder Bicyclo-(3,3,0)-octen-2 in die entsprechenden Diole bzw. Tetrole umgewandelt werden. Bei der Hydroxylierung der Diolefine werden oft sofort die entsprechenden Tetrole gebildet, auch wenn die Ameisensäure und das Wasserstoffperoxid im Unterschuß eingesetzt werden. Die Umsetzungen verlaufen überraschenderweise trotz der verhältnismäßig milden Reaktionsbedingungen sehr glatt und führen innerhalb tragbarer Reaktionszeiten zu hohen Ausbeuten an den gewünschten Diolen bzw. Tetrolen.

Bei der praktischen Durchführung des erfindungsgemäßen Verfahrens wird vorzugsweise die gesamte einzusetzende Ameisensäure zusammen mit dem Olefin vorgelegt und das Wasserstoffperoxid langsam zudosiert. Es ist jedoch auch möglich, zunächst nur einen Teil, beispielsweise etwa ein Drittel der Gesamtmenge des zu hydroxylierenden Olefins, zusammen mit der Gesamtmenge an Ameisensäure vorzulegen und das Wasserstoffperoxid und die Restmenge des zu hydroxylierenden Olefins langsam zuzudosieren. In jedem Falle wird das Reaktionsgemisch intensiv gerührt. Zur Verbesserung des Umsatzes empfiehlt sich eine angemessene Nachreaktionszeit, nachdem alle Reaktionsteilnehmer im Reaktionsgefäß vereinigt sind.

Nach beendeter Umsetzung liegen die gebildeten Diole bzw. Tetrole üblicherweise in der wäßrigen Phase gelöst vor. Für manche Zwecke können derartige wäßrige Lösungen direkt weiterverwendet werden. In den meisten Fällen wird sich aber nicht nur der Restgehalt an Wasserstoffperoxid störend auswirken, sondern es ist auch erwünscht, daß die gebildeten Diole bzw. Tetrole in reinerer Form isoliert werden. Dann kommen für die Aufarbeitung verschiedene Maßnahmen in Betracht.

Falls das eingesetzte Olefin nicht vollständig umgesetzt wurde, so daß sich aus dem Reaktionsgemisch eine zweite Phase abscheidet, wird zunächst eine Phasentrennung vorgenommen. Alternativ kann auch das nicht umgesetzte Olefin abdestilliert werden.

In vielen Fällen ist es dann ratsam, das in der wäßrigen Phase noch enthaltene Wasserstoffperoxid weitgehend zu zersetzen. Die Zersetzung kann durch längeres Stehenlassen bei höherer Temperatur (Digestion) oder durch die Einwirkung eines geeigneten Katalysators (z. B. Überleiten über einen Platin-Festbett-Kontakt) oder durch eine Kombination dieser beiden Maßnahmen bewirkt werden.

Nun wird zweckmäßigerweise das Reaktionsgemisch nach Neutralisation der enthaltenen Ameisensäure mit einem geeigneten Lösungsmittel, beispielsweise Äthylacetat, extrahiert. Aus dem Extrakt wird das Lösungsmittel abdestilliert und der verbleibende Rückstand an rohem Diol bzw. Tetrol gegebenenfalls durch Erwärmen unter vermindertem Druck von noch enthaltenem Wasser befreit. Alternativ kann auch auf die Neutralisation der im Reaktionsgemisch enthaltenen Ameisensäure verzichtet werden. Dann ist es zweckmäßig, entweder die Eindickung nach Art einer Wasserdampfdestillation kontinuierlich vorzunehmen und so lange fortzuführen, bis das Destillat nur noch geringe Mengen an Ameisensäure enthält. Oder die Ameisensäureabtreibung wird diskontinuierlich derart vorgenommen, daß man den Rückstand nach dem Abtreiben der Hauptmenge an Ameisensäure nochmals mit Wasser versetzt, erneut eindickt und diese Operation mehrmals wiederholt. Der verbleibende Rückstand an rohem Diol bzw. Tetrol kann dann wiederum durch Erwärmen unter vermindertem Druck von noch enthaltenen flüchtigen Anteilen befreit werden.

Die so erhaltenen rohen Diole bzw. Tetrole liegen mit Reinheitsgraden von etwa 88 bis 96% vor. Sofern eine noch weitergehende Reinigung erforderlich ist, kann diese in üblicher Weise durch Umkristallisation oder durch fraktionierte Destillation unter vermindertem Druck bewirkt werden.

Das erfindungsgemäße Verfahren wird durch die nachfolgenden Beispiele näher erläutert. Prozentangaben bedeuten, sofern nichts anderes angegeben ist, in allen Fällen Gewichtsprozente.

### Beispiel 1

In einer Rührapparatur mit aufgesetztem Rückflußkühler wurden 1 Mol Cyclopenten (68 g) mit 1 Mol Ameisensäure (98%ig; 47 g) vermischt und bei Siedekühlung (d. h. ca. 45°C) in 5 Stunden mit 1,5 Mol $H_2O_2$ (20%ig; 255 g) versetzt. Der Ansatz wurde danach noch weitere 3 Stunden bei 50°C gerührt und bestand dann aus einer Phase. Durch Überleiten der Flüssigkeit über ein Glasrohr, das mit einem Platin-Trägerkontakt gefüllt war, wurde das Reaktionsgemisch katalytisch von $H_2O_2$-Restmengen befreit. Mit 100 g NaOH (40%ig) wurde der Ansatz danach neutralisiert und viermal hintereinander mit je 100 ml Äthylacetat extrahiert. Aus den vereinigten und destillativ eingeengten Äthylacetat-Extrakten wurden 51 g rohes Cyclopentandiol gewonnen. Die bei der Extraktion verbleibende wäßrige Phase wurde destillativ auf ca. die Hälfte eingeengt und erneut zweimal mit je 100 ml Äthylacetat extrahiert. Beim Einengen dieser Äthylacetat-Extrakte fielen weitere 41 g rohes Cyclopentandiol an, so daß sich insgesamt eine Rohausbeute von 92 g (=90%) ergab. Das Rohdiol kann durch Umkristallisation oder durch Totaldestillation ($Kp_{12}=126-128°C$) in ein Cyclopentandiol hoher Reinheit mit einem Schmelzpunkt von 50°C überführt werden.

### Beispiel 2

In einer Apparatur gemäß Beispiel 1 wurden 1 Mol Cyclohexen (82 g) mit 0,6 Mol Ameisensäure (98%ig; 28 g) vermischt und innerhalb von 4 Stunden bei 55 bis 60°C mit 1,5 Mol $H_2O_2$ (20%ig; 255 g) versetzt. Nach weiteren 4 Stunden Nachreaktion bei 60°C blieb der Ansatz zweiphasig und es konnten 8 g Cyclohexen zurückgewonnen werden. Anschließend erfolgte eine Aufarbeitung gemäß Beispiel 1, d. h. der Ansatz wurde katalytisch von $H_2O_2$ befreit, mit 50 g NaOH (40%ig) neutral gestellt und mit Äthylacetat extrahiert. Bei der ersten Aufarbeitung der Äthylacetatextrakte fielen 75 g Rohdiol und bei der zweiten 23 g Rohdiol an. Auf den Umsatz von 74 g Cyclohexen berechnet, ergab sich somit eine Rohdiol-Ausbeute von 98 g (=94%). Das Roh-Cyclohexandiol wurde durch Totaldestillation ($Kp_{12}=119-121°C$) weiter gereinigt. Dabei fielen 84 g reines trans-Cyclohexandiol (Smp 102/3°C) an.

Der Versuch wurde wiederholt mit dem Unterschied, daß das Wasserstoffperoxid im Verlaufe von 3 Stunden bei Siedekühlung (d. h. ca. 75°C) eindosiert und die Nachreaktionszeit auf 3 Stunden verkürzt wurde. Bei der Aufarbeitung wie oben wurden praktisch die gleichen Ausbeuten erzielt.

### Beispiel 3

In einer Apparatur gemäß Beispiel 1 wurden 1 Mol Cyclohepten (96 g) mit 1 Mol Ameisensäure (98%ig; 47 g) vermischt und wie in Beispiel 1 mit $H_2O_2$ umgesetzt. Der Ansatz war nach der angegebenen Nachreaktionszeit 1phasig geworden, wurde katalytisch von $H_2O_2$ befreit, mit 80 g NaOH (40%ig) neutral gestellt und in der vorher beschriebenen Weise mit Äthylacetat aufgearbeitet.

| | |
|---|---|
| Der erste Äthylacetat-Extrakt ergab | 108 g Roh-Diol |
| Der zweite Äthylacetat-Extrakt ergab | 22 g Roh-Diol |
| Rohdiol-Ausbeute = 100% | = 130 g |

Durch Totaldestillation ($Kp_{10}$ = 129 – 131°C) wurden 108 g Cycloheptandiol (rein) gewonnen (Smp. 63/4°C; Reinausbeute 83%).

## Beispiel 4

Cyclooctadien-1,5 ist das Primärprodukt, das bei der Ringpolymerisation von Butadien anfällt.

1 Mol Cyclooctadien-1,5 (108 g) wurden in einer Apparatur gemäß Beispiel 1 mit 1 Mol Ameisensäure (98%ig; 47 g) vermischt und bei 55°C im Verlauf von 5 Stunden mit 2,66 Mol $H_2O_2$ (20%ig; 455 g) versetzt. Nachreaktion: 3 Stunden bei 60°C. Nach dieser Zeit konnten aus dem Ansatz 12 g unumgesetztes Cyclooctadien destillativ abgetrieben werden, d. h. der Dien-Umsatz betrug 89%. Daraus, sowie aus dem ermittelten hohen $H_2O_2$-Verbrauch ging hervor, daß im umgesetzten Dien beide Doppelbindungen gleichzeitig hydroxyliert worden waren.

Der Ansatz wurde katalytisch von $H_2O_2$-Restmengen befreit und mit 80 g NaOH (33%ig) neutral gestellt. Zur Erleichterung der Extraktion mit Äthylacetat wurde der Ansatz auf die Hälfte eingeengt und in üblicher Weise viermal mit je 100 ml Äthylacetat extrahiert. Das nach der Äthylacetat-Abtreibung verbleibende Sumpfprodukt wog 135 g, was einer 86%igen Rohausbeute an Cyclooctantetrol-1,2,5,6 entsprach. Eine Erhöhung der Rohausbeute kann durch erschöpfende Extraktion der wäßrigen Phase erreicht werden. Das Roh-Tetrol läßt sich im Hochvakuum ($Kp_{0.1}$ = 145 – 155°C) destillativ weiter reinigen. Es zeigte sich, daß das Cyclooctantetrol leicht mit Wasser ein Addukt gibt, das bei 83°C schmilzt.

## Beispiel 5

Cyclooctadien-1,5 läßt sich katalytisch zu Cyclooctadien-1,3 isomerisieren, das seinerseits ein im Handel erhältliches Produkt darstellt.

1 Mol Cyclooctadien-1,3 wurde analog Beispiel 4 in das Cyclooctantetrol-1,2,3,4 überführt, das ebenfalls im Hochvakuum ($Kp_{0.1}$ = 150 – 160°C) gereinigt werden kann, einen Schmelzpunkt von ca. 50°C hat und leicht ein $H_2O$-Addukt bildet.

## Beispiel 6

Cyclooctadien-1,5 kann auch durch katalytische Isomerisierung in ein anderes Handelsprodukt überführt werden, das Bicyclo-(3,3,0)-Octen-2.

Bicycloocten (1 Mol = 108 g) wurde in einer Apparatur gemäß Beispiel 1 mit 0,75 Mol Ameisensäure (98%ig; 35 g) vermischt und bei 55°C im Verlauf von 4 Stunden mit 1,3 Mol $H_2O_2$ (20%ig; 221 g) versetzt. Nachreaktion: 3 Stunden bei 60°C. Nach der katalytischen $H_2O_2$-Zersetzung und Neutralisation mit NaOH (30%ig; 95 g) ergibt der Ansatz 2 Phasen. Die wäßrige Phase wurde viermal mit je 100 ml Äthylacetat extrahiert. Die Äthylacetatextrakte wurden mit der organischen Phase vereinigt. Aus dieser Mischung schied sich eine geringe Menge einer wäßrigen Phase ab, die abgezogen wurde. Bei der Destillation der organischen Phase verblieb zunächst ein Sumpfprodukt (123 g Roh-Bicyclooctandiol; 87%ige Rohausbeute), das durch Totaldestillation bei 0.1 Torr weiter gereinigt wurde. Die bei 125 – 127°C übergehende Fraktion (111 g = 78% Reinausbeute) stellte Bicyclooctandiol (Erstarrungspunkt = 45°C) dar.

Der Versuch wurde wiederholt mit dem Unterschied, daß 0,75 Mol 20%ige Ameisensäure (175 g) und 1,3 Mol 40%iges Wasserstoffperoxid (111 g) zum Einsatz kamen. Die Aufarbeitung erfolgte wie oben, und es wurde praktisch die gleiche Ausbeute erzielt.

## Patentansprüche

1. Verfahren zur Hydroxylierung von Cyclopenten, Cyclohexen, Cyclohepten, Bicyclo-(2,2,1)-hepta-dien-2,5 oder der cyclischen Dimerisierungsprodukte von Butadien oder Isopren, sowie deren Isomerisierungsprodukte durch Umsetzung mit Ameisensäure mit einer Konzentration über 20 Gewichtsprozent und Wasserstoffperoxid mit einer Konzentration von weniger als 50 Gewichtsprozent und in einer Menge von weniger als 2 Mol pro Mol zu hydroxylierender Doppelbindung bei einer

**0 025 891**

Temperatur zwischen 40 und 80°C, dadurch gekennzeichnet, daß man das zu hydroxylierende Olefin mit weniger als 2 Mol Ameisensäure pro Mol zu hydroxylierender Doppelbindung und dem Wasserstoffperoxid umsetzt, Ameisensäure mit einer Konzentration zwischen 20 und 100 Gewichtsprozent einsetzt, und die Konzentration des Wasserstoffperoxids in der wäßrigen Phase des Reaktionsgemisches während der Gesamtdauer der Umsetzung unter 15 Gewichtsprozent hält.

2. Verfahren nach Anspruch 1, dadurch gekennzeichnet, daß man die Umsetzung bei einer Temperatur zwischen 45 und 60°C vornimmt.

3. Verfahren nach Anspruch 1 oder 2, dadurch gekennzeichnet, daß man die Ameisensäure in einer Menge von 0,2 bis 0,8 Mol pro Mol zu hydroxylierender Doppelbindung einsetzt.

## Claims

1. A process for the hydroxylation of cyclopentene, cyclohexene, cycloheptene, bicyclo-(2,2,1)-heptadiene-2,5 or the cyclic dimerisation products of butadiene or isoprene, and the isomerisation products thereof, by a reaction with formic acid having a concentration of more than 20% by weight and hydrogen peroxide having a concentration of less than 50% by weight and in a quantity of less than 2 mols per mol of double bond to be hydroxylated, at a temperature of from 40 to 80°C, characterised in that the olefin to be hydroxylated is reacted with less than 2 mols of formic acid per mol of double bond to be hydroxylated and with the hydrogen peroxide, formic acid having a concentration of from 20 to 100% by weight is used, and the concentration of the hydrogen peroxide in the aqueous phase of the reaction mixture is maintained below 15% by weight for the complete duration of the reaction.

2. A process according to claim 1, characterised in that the reaction is carried out at a temperature of from 45 to 60°C.

3. A process according to claim 1 or 2, characterised in that the formic acid is used in a quantity of from 0.2 to 0.8 mols per mol of double bond to be hydroxylated.

## Revendications

1. Procédé d'hydroxylation de cyclopentène, cyclohexène, cycloheptène, bicyclo-(2,2,1)-hepta-diène-2,5 ou des produits de dimérisation cycliques du butadiène ou de l'isoprène, ainsi que de leurs produits d'isomérisation, par réaction avec de l'acide formique à une concentration supérieure à 20% en poids et de l'eau oxygénée à une concentration inférieure à 50% en poids, et en proportion inférieure à 2 moles par mole de double liaison à hydroxyler, à une température comprise entre 40 et 80°C, procédé caractérisé en ce que l'on fait réagir l'oléfine à hydroxyler avec moins de 2 moles d'acide formique par mole de double liaison à hydroxyler et avec de l'eau oxygénée, que l'on met en oeuvre l'acide formique à une concentration de 20 à 100% en poids, et que la concentration de l'eau oxygénée dans la phase aqueuse du mélange réactionnel, pendant toute la durée de la réaction, est maintenue à moins de 15% en poids.

2. Procédé selon la revendication 1, caractérisé en ce que la réaction est effectuée à une température comprise entre 45 et 60°C.

3. Procédé selon l'une des revendications 1 ou 2, caractérisé en ce que l'on met en oeuvre l'acide formique dans la proportion de 0,2 à 0,8 mole par mole de double liaison à hydroxyler.

5